(19) **Europäisches Patentamt**

**European Patent Office**

**Office européen des brevets**

(11) **EP 0 991 877 B1**

(12) **EUROPÄISCHE PATENTSCHRIFT**

(45) Veröffentlichungstag und Bekanntmachung des Hinweises auf die Patenterteilung:
**23.01.2002 Patentblatt 2002/04**

(51) Int Cl.⁷: $F16H\ 21/52$, $F04B\ 9/02$, $F04B\ 43/02$

(21) Anmeldenummer: **98941222.6**

(22) Anmeldetag: **15.06.1998**

(86) Internationale Anmeldenummer:
**PCT/DE98/01705**

(87) Internationale Veröffentlichungsnummer:
**WO 98/59187 (30.12.1998 Gazette 1998/52)**

(54) **VORRICHTUNG, INSBESONDERE PUMPE**

DEVICE, SPECIALLY A PUMP

DISPOSITIF, EN PARTICULIER UNE POMPE

(84) Benannte Vertragsstaaten:
**AT BE CH DE DK ES FI FR GB GR IE IT LI LU NL PT SE**

(30) Priorität: **24.06.1997 DE 19726702**

(43) Veröffentlichungstag der Anmeldung:
**12.04.2000 Patentblatt 2000/15**

(73) Patentinhaber:
• **Kaufmann, Ralf**
  **52072 Aachen (DE)**
• **Nix, Christoph**
  **52076 Aachen (DE)**
• **Rau, Günter**
  **52066 Aachen (DE)**
• **Reul, Helmut, Prof. Dr.**
  **52353 Düren (DE)**

(72) Erfinder:
• **Kaufmann, Ralf**
  **52072 Aachen (DE)**
• **Nix, Christoph**
  **52076 Aachen (DE)**
• **Rau, Günter**
  **52066 Aachen (DE)**
• **Reul, Helmut, Prof. Dr.**
  **52353 Düren (DE)**

(74) Vertreter: **Castell, Klaus, Dr.**
  **Gutenbergstrasse 335**
  **52349 Düren (DE)**

(56) Entgegenhaltungen:
**EP-A- 0 077 087        EP-A- 0 728 489**
**EP-A- 0 768 408        DE-A- 3 811 828**
**US-A- 4 512 726**

**Beschreibung**

Die Erfindung betrifft eine Vorrichtung, insbesondere eine Pumpe, zur Umsetzung einer gleichgerichteten Drehbewegung in eine Hin- und Herbewegung.

Für derartige Aufgaben ist vor allem eine Exzenterscheibe bekannt, deren Exzenter in einem Langloch eines lateral verschiebbar geführten Teiles sitzt, wobei durch die Drehung der Exzenterscheibe das lateral geführte Element in Form einer Sinusbewegung hin- und her wandert.

Aus der DE-A- 3 811 828 und der EP-A- 0 768 408 sind Vorrichtungen bekannt, bei denen ein gekröpftes Element verwendet wird. Diese Vorrichtungen haben Achsen, die sich aus kinematischen Gründen in einem Raumpunkt schneiden. Aus baulichen Gründen kann dieser Schnittpunkt bei der Verwendung eines gekröpften Elementes nicht im Bereich des gekröpften Elementes liegen. Dadurch entsteht ein relativ großer Bauraum, der für viele Anwendungen nachteilhaft ist. Die bekannten Vorrichtungen sind zwar gut dazu geeignet, schnelle, durch Schwungmassen stabilisierte Rotationsbewegungen in relativ kleine Hin- und Herbewegungen umzuwandeln. Insbesondere, wenn ein großer Hub und zusätzlich eine relativ langsame Drehbewegung mit relativ großen Momenten erzeugt werden soll, sind die bekannten Einrichtungen jedoch ungeeignet.

Der Erfindung liegt daher die Aufgabe zugrunde, eine neue Vorrichtung der eingangs beschriebenen Art vorzustellen, die vor allem nur eine sehr begrenzte Bauhöhe benötigt.

Diese Aufgabe wird mit einer Vorrichtung gelöst, die folgendes aufweist: Eine Scheibe, die um eine zentrale Achse drehbar ist, einen Taumelbolzen, der um eine in einem Winkel zur Scheibenebene angeordnete Achse drehbar in der Scheibe gelagert ist, einen Schwenkbolzen, der in einem Winkel zur Achse des Taumelbolzens im Taumelbolzen befestigt ist, und ein Haltemittel, das den Schwenkbolzen von einer Drehbewegung um die Achse der Scheibe abhält.

Diese Vorrichtung erlaubt es, die um die zentrale Achse drehbare Scheibe kontinuierlich zu drehen, um den Schwenkbolzen hin- und herwandern zu lassen. Durch die Wahl der Winkel und Längen der verwendeten Mittel ist die Amplitude der Hin- und Herbewegung beliebig variierbar.

Während die Scheibe extrem dünn gehalten werden kann, legt der Winkel des Taumelbolzens und der Winkel und die Länge des Schwenkbolzens die Höhe und gleichzeitig die Amplitude der Vorrichtung fest.

Der Winkel zwischen Schwenkbolzen und Taumelbolzen ist vorteilhafterweise fest zwischen 45° und 90° eingestellt und in einer bevorzugten Ausführungsform ist der gleiche Winkel zwischen Taumelbolzen und Scheibenebene vorgesehen.

Vorteilhaft ist es, wenn das Haltemittel an einem die Scheibe umgebenden Gehäuse befestigt ist. Da das Gehäuse relativ zur Scheibe stationär angeordnet ist, erlaubt eine Befestigung des Haltemittels am Gehäuse ein Halten des Schwenkbolzens relativ zur Scheibe und somit die Erzeugung der auf einem Halbkreis senkrecht zur Scheibe hin- und hergehenden Bewegung des Schwenkbolzens.

Ein einfacher Aufbau des Haltemittels sieht vor, daß es an einem ortsfesten Schwenkarmbolzen und dem Schwenkbolzen drehbar gelagert ist. Dies erlaubt eine spielfreie Führung des Schwenkbolzens und somit eine präzise Ausführung der Bewegung.

Ein leicht konstruierbarer Aufbau wird dadurch erreicht, daß das Lager des Schwenkarmbolzens und das Lager des Schwenkbolzens orthogonal zueinander angeordnet sind.

Um die auf dem Halbkreisbogen ausgeführte Hin- und Herbewegung des Schwenkbolzens in eine laterale Bewegung zu überführen wird vorgeschlagen, daß der Schwenkbolzen an seinem dem Taumelbolzen gegenüberliegenden Ende mindestens eine Rolle aufweist, die mit einer in Richtung der zentralen Achse der Scheibe beweglichen Druckplatte zusammenwirkt. Die Druckplatte kann dabei eine Führung für die Rolle aufweisen, die bei drehfest angeordneter Platte als Haltemittel dienen kann.

Eine besonders einfache Ausgestaltung, insbesondere für die Verwendung der Vorrichtung als Pumpe, sieht vor, daß die Bewegung der Druckplatte von einer Membran geführt ist. Die Membran erlaubt eine Bewegung der Druckplatte in einem bestimmten Bereich und kann gleichzeitig als Abdichtung für eine als Kolben wirkende Druckplatte dienen.

Eine vorteilhafte Ausgestaltung der Erfindung sieht vor, daß die Membran am Gehäuse befestigt ist. Somit wird einerseits der Raum der beweglichen Teile abgedichtet und andererseits eine einfache Befestigung der Membran erzielt.

Vorteilhaft ist es, wenn die Membran mit einem Gehäuseteil eine Pumpenkammer begrenzt. Dies erlaubt einen kompakten Aufbau einer als Pumpe ausgebildeten Vorrichtung, wobei die Pumpe mit Antrieb und Umsetzungsmechanik eine extrem geringe Bauhöhe aufweist.

Eine bevorzugte Verwendung sieht vor, daß die Vorrichtung zum Pumpen von Blut verwendet wird. Die Vorrichtung kann durch ihre geringe Bauhöhe nicht nur angenehm am Körper getragen werden, sondern ist wegen ihrer niedrigen Bauhöhe auch zur Implantation in den Thorax geeignet.

Die beschriebene Vorrichtung eignet sich vor allem für implantierbare pulsierend fordernde Herzunterstützungssysteme, die auch bei Kindern und kleinwüchsigen Erwachsenen einsetzbar sind. Die Vorrichtung ist sowohl implantierbar als auch extrakorporal einsetzbar.

Die Vorrichtung eignet sich aber allgemein zur Umwandlung einer umlaufenden Drehung mit dem Winkel in eine orthogonal schwingende Drehung mit dem Winkel $\psi$ und ist vor allem zum Herausklappen von technischen Strukturen aus sehr flachen Gehäusen oder Rahmenstrukturen wie beispielsweise Bremsklappen an Flugzeugtragflächen, Fahrgestelle aus dünnwandigen Strukturen etc. geeignet.

Ein weiterer Vorteil der erfindungsgemäßen Vorrichtung ist die selbsthemmende Winkelverstellung von technischen Strukturen, die einen Einsatz beispielsweise bei Gliedern von Robotern, Rückenlehnen von Sitzen, Kfz-Rückspiegeln, Leitblechen in Distributionssystemen, Lüftungsklappen, Schiffsruderanlagen u. ä. begünstigen, da die Vorrichtung bei Stromausfall in der vorgegebenen Position verharrt.

Bei kleinen Stellwinkelbereichen $\psi$ sind - wenn erforderlich - sehr hohe Kraftuntersetzungen möglich und die Vorrichtung läßt sich zu den oben genannten Zwecken leicht manuell oder fernmotorisch über Zahnriemen, Keilriemen oder Gelenkgestelle betätigen.

Ein Ausführungsbeispiel der erfindungsgemäßen Vorrichtung ist in der Zeichnung dargestellt und wird im folgenden näher beschrieben.

Es zeigt,

Figur 1     eine als Blutpumpe ausgebildete Vorrichtung, die im Thorax zwischen dem Brustkorb und dem linken Lungenflügel implantiert ist,

Figur 2     eine perspektivische Darstellung der Vorrichtung mit weggebrochenem Gehäuseteil und abgehobener Pumpenkammer,

Figur 3     einen Schnitt durch die erfindungsgemäße Vorrichtung nach Ausstoß des zu fördernden Blutvolumens,

Figur 4     einen Schnitt durch die Vorrichtung nach Figur 3 während der Füllung mit Blut aus dem Herzventrikel,

Figur 5     die Scheibe, den Taumelbolzen und den Schwenkbolzen der Vorrichtung nach den Figuren 1 - 4,

Figur 6     ein Diagramm mit der Übertragungsfunktion des Hubgetriebes und

Figur 7     eine Scheibe einer alternativen Vorrichtung mit zwei Taumelbolzen und jeweils einem daran befestigten Schwenkarm.

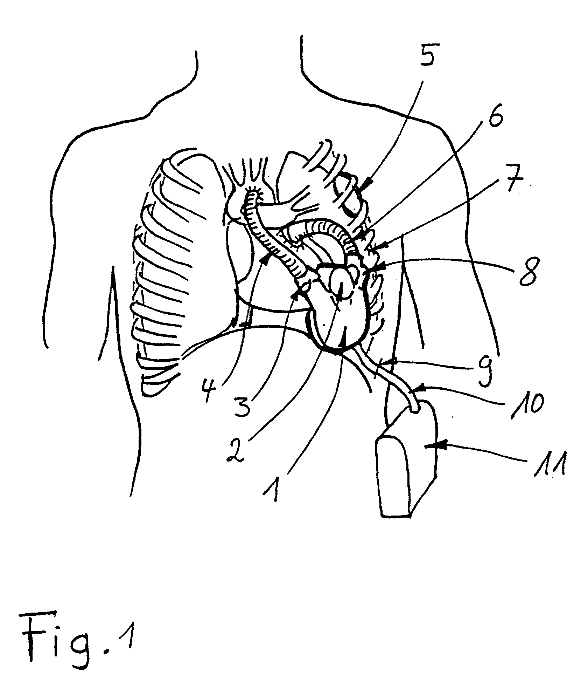

Figur 1 zeigt ein Blutpumpengehäuse 1 im implantierten Zustand zwischen Brustkorb und linkem unteren Lungenlappen im Thorax. Der elektrische Antriebsmotor 2 befindet sich innerhalb des Gehäuses 1 zwischen dem Pumpeneinlaßventil 8 und dem Pumpenauslaßventil 3.

Das Auslaßventil 3 ist durch eine Anschlußkanüle 4 mit der Aorta zur Entlastung des linken Herzventrikels verbunden. Sofern eine Entlastung des rechten Ventrikels gewünscht ist, wird die Anschlußkanüle 4 mit der Pulmonalarterie verbunden. Das Einlaßventil 8 ist mittels einer Anschlußkanüle 6 durch den Vorhof mit dem dazugehörigen Ventrikel verbunden.

Das Gasvolumen im Blutpumpengehäuse 1 ist durch einen Schlauch 7 mit einem implantierten Volumenkompensator 5 verbunden. Zur Konstanthaltung des Gasdruckes im Blutpumpengehäuse 1 gleicht der Volumenkompensator 5 die Schwankungen des Gasvolumens aus, die durch die Volumenänderung der Pumpenkammer bei der Pumpaktion (Füllen und Ausstoßen) hervorgerufen werden.

Die Energieversorgung und Zuführung von Regelungssignalen erfolgt über ein Kabel 10, das durch einen kleinen Hautschnitt 9 die Verbindung zur extrakorporalen Batterie-, Leistungs-, und Regelungselektronikeinheit 11 bildet.

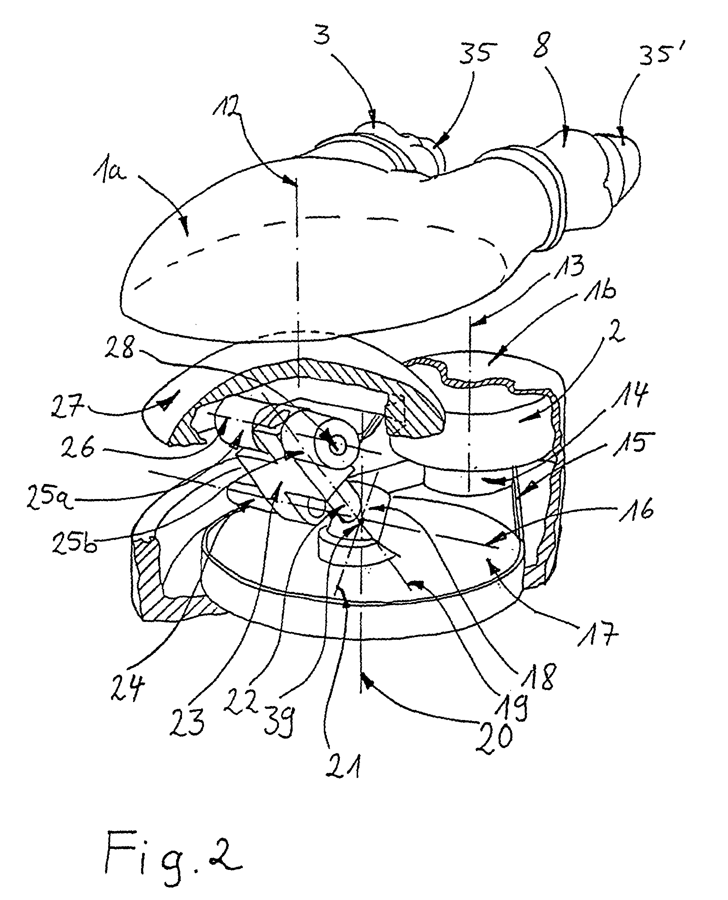

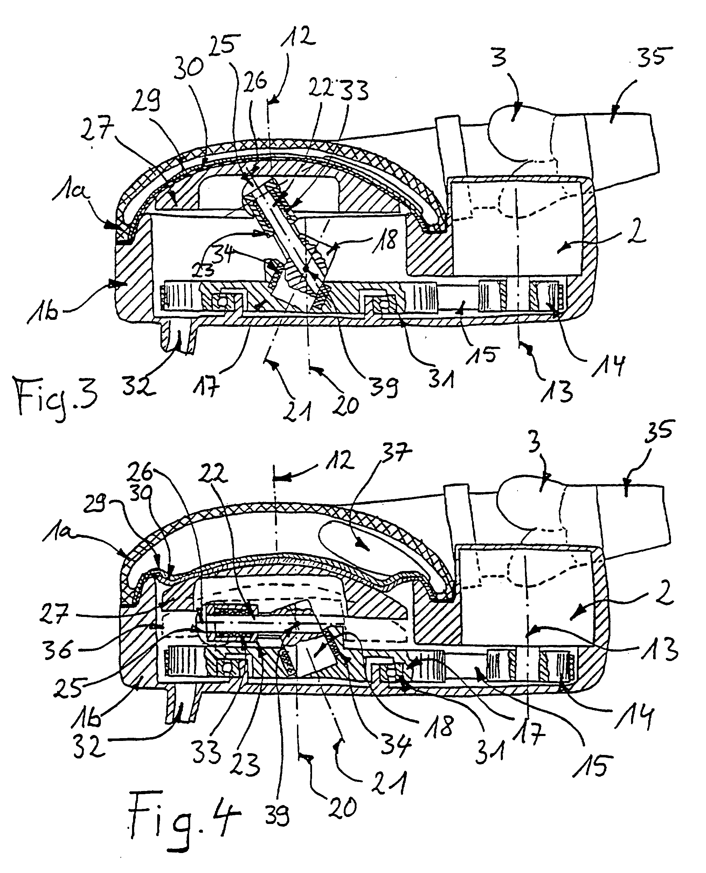

Figur 2 zeigt perspektivisch das Innere der Blutpumpe. Der vordere Teil des Gehäuseunterteils 1b ist dabei weggeschnitten und das Pumpkammergehäuse Teil 1a ist entlang der Pumpkammermittelachse 12 nach oben verschoben dargestellt. Am Pumpkammergehäuse 1a ist das Auslaßventil 3 und das Einlaßventil 8 mit je einem Kanülenanschlußstutzen 35, 35' angebracht. Ein Elekromotor 2 im Gehäuseunterteil 1b treibt gleichförmig und gleichgerichtet mit einem Ritzel über einen Zahnriemen 15 die Scheibe 17 an. Als Alternative ist auch ein Antrieb mit einer Zahnradübertragung zwischen dem Ritzel 14 und der Scheibe 17 möglich. Die Mittelachse 13 des Elektromotors ist parallel zur Rotationsachse 20 der Scheibe 17 angeordnet.

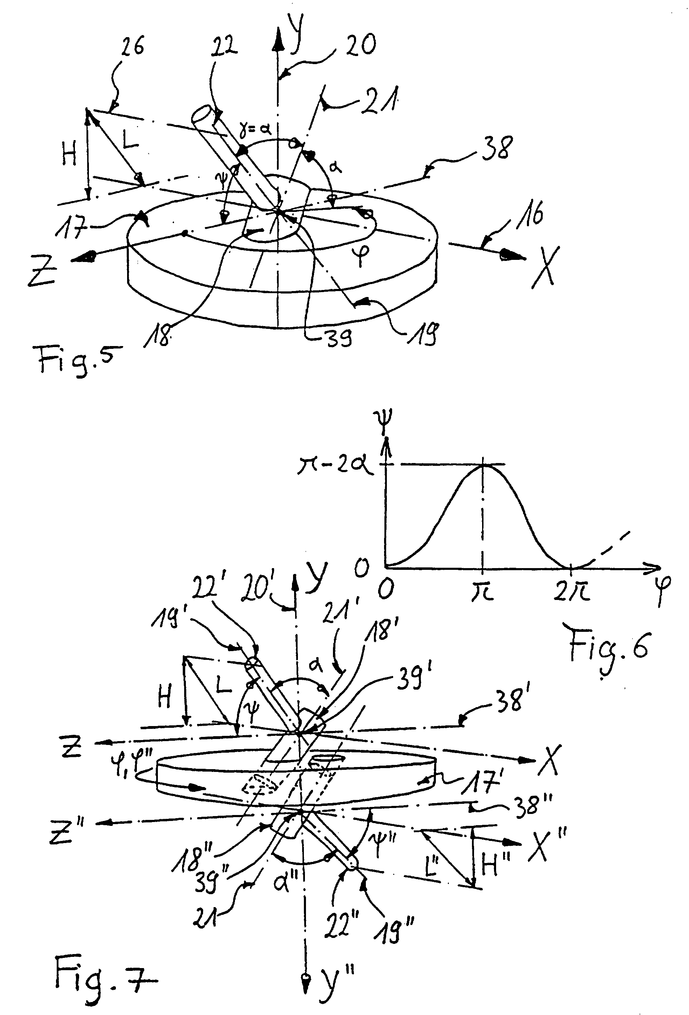

Im Zentrum der Scheibe 17 befindet sich eine zur Scheibenebene schräg eingebrachte Bohrung, in der ein Taumelbolzen 18 drehbar gelagert ist. Der Winkel $\alpha$ zwischen der Taumelbolzenachse 21 und der Scheibenebene ist kinematisch bedingt auf einen Wert zwischen $\pi/4$ und $\pi/2$ festzulegen (vgl. Figur 5).

In den Taumelbolzen 18 ist ein Schwenkbolzen 22 in einem Winkel $\gamma$ zur Achse 21 des Taumelbolzens 18 fest eingepreßt. Im vorliegenden Ausführungsbeispiel entspricht der Winkel $\gamma$ zwischen der Achse 21 des Taumelbolzens 18 und der Achse 19 des Schwenkbolzens 22 dem Winkel $\alpha$ zwischen der Achse 21 des Taumelbolzens 18 und der Scheibenebene (vgl. Figur 5). Außerdem schneiden sich die Mittelachse 19 des Schwenkbolzens 22, die Mittelachse 21 des Taumelbolzens 18 und die Rotationsachse 20 der Antriebsscheibe 17 in einem Punkt 39.

Im Gehäuseunterteil 1b ist ein Schwenkarmbolzen 24 parallel zur Ebene der Scheibe verlaufend eingepreßt. Die Mittelachse 16 dieses Schwenkarmbolzens 24 schneidet die Achsen 19, 20 und 21 ebenfalls im Punkt 39.

Der Schwenkbolzen 22 ist durch einen Schwenkarm 23 mit dem Schwenkarmbolzen 24 gekoppelt. Der Schwenkarm 23 enthält zwei orthogonal orientierte Radiallagerungen zur Aufnahme des Schwenkbolzens 22 bzw. des Schwenkarmbolzens 24. Hauptaufgabe des Schwenkarmes 23 ist es, eine Rotation des Schwenkbolzens 22 um die Achse 20 zu unterbinden.

Am Schwenkarm 23 befinden sich entlang einer Mittelachse 26 im Abstand L parallel zur Achse 16 zwei Hubwalzen 25a und 25b. Diese Hubwalzen 25a und 25b sind drehbar auf Wellen 28 befestigt. Die Wellen 28 sind fest mit dem Schwenkarm verbunden.

Während der Ausstoßphase heben die Hubwalzen 25a und 25b eine Membran geführte Druckplatte 27 entlang der Pumpkammermittelachse 12 an. Dabei rollen die Hubwalzen 25 in einer Aussparung unterhalb der Druckplatte 27 ab.

Die Figuren 3 und 4 zeigen die Blutpumpe aus Figur 2 in einem Schnitt, in dem die Motorachse 13, die Rotationsachse 20 der Scheibe 17 und die Pumpenkammermittelachse 12 liegen. In diesem Schnitt ist das Auslaßventil 3 mit seinem Kanülenstutzen 35 sichtbar.

Figur 3 zeigt die Blutpumpe nach dem Ausstoß des zu fördernden Blutvolumens. Die am Schwenkarm 23 befestigten Hubwalzen 25 sind um den Punkt 39 mit dem Winkel $\psi$-max (vgl. Figur 5) in ihre obere Endstellung geschwenkt und haben die rotationsellipsoidisch geformte Druckplatte 27 entlang der Pumpkammermittelachse 12 gegen die blutseitige Blutkammermembran 29 gedrückt und dadurch den Inhalt des Pumpkammergehäuseteils 1a entleert.

Die Druckplatte 27 ist im Zentrum mit der antriebsseitigen Führungsmembran 30 fest verbunden. Am Umfang weist die Führungsmembran 30 eine flexible Rollfalte auf. Die Führungsmembram 30 ist am Pumpkammergehäuse 1a befestigt. Die Bewegungsrichtung der Druckplatte 27 wird durch die Führungsmembran 30 hinreichend genau auf die Pumpkammermittelachse 12 beschränkt.

Die Scheibe 17 ist mit einer Axial-Radiallagerung 31 am Gehäuseunterteil 1b gelagert, der Taumelbolzen 18 ist mit einer Radiallagerung 34 in der Antriebsscheibe 17 gelagert und der Schwenkbolzen 22 ist mit einer Radiallagerung 33 im Schwenkarm 23 gelagert.

Figur 4 zeigt die Blutpumpe während sie sich - hervorgerufen durch den venösen Blutdruck - passiv mit einem neuen Blutfördervolumen füllt. Die Verdrängungswirkung des einströmenden Blutvolumens drückt die Druckplatte 27-geführt durch die Führungsmembran 30- entlang der Pumkammermittelachse 12 in die untere Endstellung, die durch eine gestrichelt dargestellte Druckplatte 36 angedeutet ist.

Die Scheibe 17 hat sich gegenüber der Situation in Figur 3 um 180° gedreht und dadurch den Taumelbolzen 18 gekippt. Der Schwenkbolzen 22 ist in seine untere Stellung geschwenkt und hat über seine Radiallagerung 33 den Schwenkarm 23 mit den dort befestigten Hubwalzen 25 mitgenommen.

Während der Füllung des Pumpkammergehäuses 1a mit Blut entweicht das verdrängte Gas aus dem Getrieberaum über einen Schlauchstutzen 32 in den Volumenkompensator 5, der in Figur 1 abgebildet ist.

Nach einer weiteren Drehung der Scheibe 17 um 180° haben die durch den beschriebenen Mechanismus angetriebenen Hubwalzen 25 die Druckplatte 27 wieder nach oben gedrückt und dadurch das Pumpkammergehäuse 1a über eine Öffnung 37 durch das Auslaßventil 3 erneut entleert (vgl. Figur 3).

In Figur 5 sind in einer schematischen Darstellung der Scheibe 17 mit Taumelbolzen 18 und Schwenkbolzen 22 die kinematischen Parameter $\alpha$, $\varphi$, $\psi$, $\gamma$, L und H des Hubmechanismus dargestellt. Diese Parameter lassen sich in einem kartesischen Koordinatensystem beschreiben, dessen Zentrum im Punkt 39 liegt. Die X - Abzisse dieses Koordinatensystems ist identisch mit der Mittelachse 16 des Schwenkarmbolzens 24 (nicht gezeigt) und weist in positiver Richtung von seiner Befestigung am Gehäuseunterteil 1b weg. Die Y - Achse ist identisch mit der Rotationsachse 20 der Scheibe 17 und weist in positiver Richtung zum Pumpkammergehäuse 1a hin. Die Z-Achse 38 weist in positiver Richtung vom Motor 2 weg.

Der Winkel $\alpha$ liegt zwischen der Mittelachse 21 des Taumelbolzens 18 und der X-Z-Ebene und entspricht dem Winkel $\gamma$ zwischen der Mittelachse 21 des Taumelbolzens 18 und der Mittelachse 19 des Schwenkbolzens 22. Dieser Winkel $\alpha$ liegt zwischen $\pi/4$ und $\pi/2$.

$\varphi$ ist der Drehwinkel der Scheibe 17 bzw. der Mittelachse 21 des Taumelbolzens 18 um die Y - Achse. Er wird von der positiven Z-Achse aus gegen den Uhrzeigersinn aufgetragen.

$\psi$ ist der Schwenkwinkel des Schwenkarms 22 zur Z-Achse.

Nach einer halben Umdrehung der Scheibe 17 ($\varphi = \pi$) erreicht der Schwenkwinkel $\psi$ seinen Maximalwert.

$$\psi_{max} = \pi - 2\cdot\alpha \qquad (\text{wenn } \alpha = \gamma)$$

Nach einer weiteren halben Umdrehung ($\varphi = 2\pi$) geht der Schwenkwinkel $\psi$ auf 0 zurück.

L ist der Abstand zwischen der X-Achse und der zu ihr parallelen Mittelachse 26 der Wellen 28. H ist der Hubraum und somit die Höhe der Mittelachse 26 über der X-Z-Ebene. Es ist:

$$H = L \cdot \text{sinus } \psi.$$

In Figur 6 ist die Übertragungsfunktion des Getriebes mit dem Drehwinkel $\varphi$ der Scheibe 17 als Eingangsgröße und dem Schwenkwinkel $\psi$ des Schwenkbolzens 22 als Ausgangsgröße dargestellt. Die Übertragungsfunktion lautet:

$$\psi = \arccos (D + (D^2 - (1 - \tan^2 \alpha)/ (\cos^2 \varphi + \tan^2 \alpha))^{0.5})$$

mit

$$D = \cos\varphi / (\cos^2\varphi + \tan^2 \alpha) \qquad (\text{wenn } \alpha = \gamma).$$

Die in Figur 6 dargestellte Übertragungsfunktion wird von einem flachen Getriebe erzeugt, das den Bau einer sehr flachen, pulsierend fördernden Blutpumpe ermöglicht, die intrathorakal zwischen Lungen- und Rippenfell unter Vermeidung unzulässiger Gewebekompression implantiert werden kann. Der Unterdruck im Thorax unterstützt bei einer derartigen Blutpumpe die freie Kammerfüllung und erleichtert deren Volumenkompensation.

Figur 7 zeigt eine Alternative zum kinematischen Schema eines Hubgetriebes nach Figur 5. Dieses Hubgetriebe eignet sich für eine Blutpumpe mit zwei gegenüberliegenden Pumpkammern, wie dies für eine biventrikuläre Herzunterstützung oder ein künstliches Herz erforderlich ist. Aus Gründen der Übersichtlichkeit sind auch hier, wie in Figur 5, die Schwenkarme, Pumpkammern und alle weiteren Antriebs- und Gehäuseteile nicht eingezeichnet. Die den in den Figuren 1 - 6 entsprechenden Teile sind mit gestrichenen Bezugszeichen bezeichnet und die diesen Teilen gegenüberliegenden Teile sind mit doppelt gestrichenen Bezugsziffern bezeichnet.

Auf der einen Seite der Scheibe 17' befindet sich der Taumelbolzen 18' mit dem Schwenkbolzen 22' und auf der anderen Seite der Scheibe 17' ist ein Taumelbolzen 18" mit einem Schwenkbolzen 22" punktsymmetrisch zum Mittelpunkt der Scheibe 17' angeordnet.

Die Mittelachse 19" des Schwenkbolzens 22", die Mittelachse 21" des Taumelbolzens 18" und die Y-Achse schneiden sich im Punkt 39". In der abgebildeten Darstellung ist der Punkt 39" Mittelpunkt eines weiteren kartesischen Koordinatensystems mit den Achsen X", Y" und Z", in dem die in Figur 5 beschriebenen Parameter ($\alpha$, $\varphi$, $\psi$, L und H) ihre Entsprechung in ($\alpha$", $\varphi$", $\psi$", L" und H") finden. Hierbei ist anzumerken, daß die Zahlenwerte der ungestrichenen, gestrichenen und doppelt gestrichenen Parameter durchaus unterschiedlich sein können. Die in Figur 6 dargestellte Übertragungsfunktion ist auf die Parameter der Figur 7 entsprechend anwendbar.

Die X"- Achse liegt parallel zur X-Achse und ist identisch mit der Mittelachse 16" des zweiten Schwenkarmbolzens und weist in positiver Richtung von seiner Befestigung am Gehäuseunterteil 1b weg.

Die Y" - Achse ist hier identisch mit der Y - Achse, jedoch mit einem anderen Vorzeichen und einem verschobenen 0-Punkt. Sie ist identisch mit der Rotationsachse 20' der Scheibe 17' und weist in positiver Richtung zu einem gegenüberliegenden Pumpkammergehäuse hin (nicht gezeigt). Die Z" - Achse 38" ist parallel zur Z - Achse 38' und weist in positiver Richtung vom Motor (nicht gezeigt) weg.

Die in den Figuren 2 - 6 gezeigte Vorrichtung wirkt als Getriebe und wandelt eine gleichförmige und gleichgerichtete Rotorbewegung eines Antriebsmotors 2 in einen ozillierenden Druckplatten Hub H um. Das erweiterte Getriebe in der Ausführungsform nach Figur 7 wandelt eine gleichförmige und gleichgerichtete Rotorbewegung eines Antriebsmotors 2 in zwei ozillierende Druckplattenhübe H, H" um.

Die gleichgerichtete und gleichmäßige Rotation des Antriebs 2 vermeidet Wirkungsgrad und Wärmeverluste durch Umsteuerungselektroniken und Massenträgheitseffekte. Darüber hinaus bieten die beschriebenen Vorrichtungen bei gleichem Bauraum eine kürzere Energiewandlungskette als bei elektrohydraulischen Systemen.

Alle Getriebeelemente sind an ihren Gelenken ausschließlich durch Drehgelenke, Kugel- und Gleitlagerungen miteinander verbunden. Lineare Gleitführungen sind nicht vorhanden. Die Vorrichtung ist daher extrem verschleißarm gebaut und weist einen hohen systemimmaneten Wirkungsgrad auf. Dies ist vor allem im medizinischen Sektor, wie beispielsweise bei implantierten Blutpumpen von größter Bedeutung.

Die gleichförmige und gleichgerichtete Motorantriebsbewegung ermöglicht zusätzlich die Analyse des zyklischen Stromverbrauchs, um Aussagen über den Füllungsgrad der Pumpkammer und der Nachlastdrücke zu gewinnen. Drucksensoren sind für diesen Überwachungszweck nicht erforderlich.

Die Transmission der Rotation des Ritzels 14 auf das Antriebsrad 17 über einen Riemen 15 oder ein Zahnrad (nicht gezeigt), ermöglicht für den Motor 2 günstige Drehzahluntersetzungen.

Der Hubweg H, H' des Getriebes läßt sich konstruktiv in weiten Bereichen durch Veränderung des Parameters L oder Winkels $\alpha$ (Figur 5) variieren. Zusätzlich bietet der Gestaltungsspielraum der Druckplatte 27 gute Anpassungsmöglichkeiten an verschiedenartige Pumpkammergeometrien. Gleiches gilt auch für die Ausführungsform nach Figur 7.

Das Hubgetriebe läßt sich einfach und sicher zusammenbauen. Ein Getriebefreiheitsgrad von 1 ist durch die kinematischen Getriebeachsen 16, 19, 20 und 21 bedingt. Der Zusammenhalt der Hubgetriebeteile ist durch die axiale Fixierung des Schwenkarmbolzens 24 im Gehäuse 1 gewährleistet. Der Taumelbolzen 18 mit eingepreßtem Schwenkbolzen 22 braucht nicht axial fixiert zu sein. Somit werden bei den Ausführungsformen nach den Figuren 2 - 5 und 7 nur wenige Axiallager benötigt.

## Patentansprüche

1. Vorrichtung, insbesondere Pumpe, zur Umsetzung einer gleichgerichteten Drehbewegung in eine Hin- und Herbewegung, mit

   einer Scheibe (17), die um eine zentrale Achse (20) drehbar ist,

   einem Taumelbolzen (18), der um eine in einem Winkel $\alpha$ zur Scheibenebene angeordnete Achse (21) drehbar in der Scheibe (17) gelagert ist,

   einem Schwenkbolzen (22), der in einem Winkel $\gamma$ zur Achse (21) des Taumelbolzens (18) im Taumelbolzen (18) befestigt ist und

   einem Haltemittel (23), das den Schwenkbolzen (22) von einer Drehbewegung um die Achse (20) der Scheibe (17) abhält.

2. Vorrichtung nach Anspruch 1, *dadurch gekennzeichnet, daß* der Winkel $\gamma$ zwischen dem Schwenkbolzen (22) und dem Taumelbolzen (18) fest zwischen 45° und 90° eingestellt ist.

3. Vorrichtung nach einem der vorhergehenden Ansprüche, *dadurch gekennzeichnet, daß* der Winkel $\gamma$ zwischen Schwenkbolzen (22) und Taumelbolzen (18) dem Winkel $\alpha$ zwischen dem Taumelbolzen (18) und der Scheibenebene entspricht.

4. Vorrichtung nach einem der vorhergehenden Ansprüche, *dadurch gekennzeichnet, daß* das Haltemittel (23) an einem die Scheibe (17) umgebenden Gehäuse (1a, 1b) befestigt ist.

5. Vorrichtung nach einem der vorhergehenden Ansprüche, *dadurch gekennzeichnet, daß* das Haltemittel (23) an einem ortsfesten Schwenkarmbolzen (24) und dem Schwenkbolzen (22) drehbar gelagert ist.

6. Vorrichtung nach Anspruch 5, *dadurch gekennzeichnet, daß* das Lager des Schwenkarmbolzens (24) und das Lager des Schwenkbolzens (22) orthogonal zueinander angeordnet sind.

7. Vorrichtung nach einem der vorhergehenden Ansprüche, *dadurch gekennzeichnet, daß* der Schwenkbolzen (22) an seinem dem Taumelbolzen (18) gegenüberliegenden Ende mindestens eine Rolle (25a, 25b) aufweist, die mit einer mit Richtung der zentralen Achse (20) der Scheibe (17) beweglichen Druckplatte (27) zusammenwirkt.

8. Vorrichtung nach Anspruch 7, *dadurch gekennzeichnet, daß* die Bewegung der Druckplatte (27) von einer Membran (30) geführt ist.

9. Vorrichtung nach Anspruch 8, *dadurch gekennzeichnet, daß* die Membran (30) an einem Gehäuse (1a, 1b) befestigt ist.

10. Vorrichtung nach Anspruch 9, *dadurch gekennzeichnet, daß* die Membran (30) mit einem Gehäuseteil (1a) eine Pumpenkammer begrenzt.

**11.** Verwendung der Vorrichtung nach einem der Ansprüche 1 - 10 zum Pumpen von Blut.

## Claims

**1.** Device, particularly a pump, for converting a rotational movement in one direction into a back and forth movement, having

a disk (17) which can be rotated around a central axis (20),

a wobble pin (18) which is mounted in the disk (17) so it is rotatable around an axis (21) located at an angle a to the plane of the disk,

a swivel pin (22) which is attached in the wobble pin (18) at an angle ã to the axis (21) of the wobble pin (18), and

a support element (23), which prevents rotational movement of the swivel pin (22) around the axis (20) of the disk (17).

**2.** Device according to claim 1, **characterized in that** the angle ã between the swivel pin (22) and the wobble pin (18) is permanently set between 45° and 90°.

**3.** Device according to one of the preceding claims, **characterized in that** the angle ã between the swivel pin (22) and wobble pin (18) corresponds to the angle á between the wobble pin (18) and the plane of the disk.

**4.** Device according to one of the preceding claims, **characterized in that** the support element (23) is attached to a housing (1a, 1b) surrounding the disk (17).

**5.** Device according to one of the preceding claims, **characterized in that** the support element (23) is rotatably mounted on a fixed swivel arm pin (24) and the swivel pin (22).

**6.** Device according to claim 5, **characterized in that** the bearing of the swivel arm pin (24) and the bearing of the swivel pin (22) are positioned orthogonally to one another.

**7.** Device according to one of the preceding claims, **characterized in that** the swivel pin (22) has a roller (25a, 25b) on its end lying opposite to the wobble pin (18) which interacts with a pressure plate (27), which is movable in the direction of the central axis (20) of the disk (17).

**8.** Device according to claim 7, **characterized in that** the movement of the pressure plate (27) is guided by a membrane (30).

**9.** Device according to claim 8, **characterized in that** the membrane (30) is attached to a housing (1a, 1b).

**10.** Device according to claim 9, **characterized in that** the membrane (30) delimits a pump chamber with a housing (1a).

**11.** Use of the device according to one of the claims 1-10 for pumping blood.

## Revendications

**1.** Dispositif, en particulier une pompe, pour transformer un mouvement de rotation rectifié en un mouvement de va-et-vient, avec

un disque (17) qui peut tourner autour d'un arbre central (20),

un axe oscillant (18) qui est monté à rotation dans le disque (17) autour d'un arbre (21) disposé selon un angle α par rapport au plan du disque,

un axe pivotant (22) qui est fixé dans l'axe oscillant (18) selon un angle γ par rapport à l'arbre (21) de l'axe oscillant (18) et

un moyen d'arrêt (23) qui empêche l'axe pivotant (22) d'effectuer in mouvement de rotation autour de l'arbre (20) du disque (17).

2. Dispositif selon la revendication 1, **caractérisé en ce que** l'angle γ
entre l'axe pivotant (22) et l'axe oscillant (18) est ajusté fixement entre 45° et 90°.

3. Dispositif , selon l'une quelconque des revendications précédentes, **caractérisé en ce que** l'angle γ entre l'axe pivotant (22) et l'axe oscillant (18) correspond à l'angle α entre l'axe oscillant (18) et le plan du disque.

4. Dispositif , selon l'une quelconque des revendications précédentes, **caractérisé en ce que** le moyen d'arrêt (23) est fixé à un boîtier (1a, 1b) entourant le disque (17).

5. Dispositif , selon l'une quelconque des revendications précédentes, **caractérisé en ce que** le moyen d'arrêt (23) est monté à rotation sur un axe de bras pivotant (25) fixe et l'axe pivotant (22).

6. Dispositif selon la revendication 5, **caractérisé en ce que** le palier de axe de bras pivotant (24) et le palier de l'axe pivotant (22) sont disposés orthogonalement l'un à l'autre.

7. Dispositif , selon l'une quelconque des revendications précédentes, **caractérisé en ce que** l'axe pivotant (22) présente au niveau de son extrémité opposée à l'axe oscillant (18) au moins un rouleau (25a, 25b) qui agit de concert avec une plaque d'appui (27) mobile dans le sens de l'arbre central (20) du disque (17).

8. Dispositif selon la revendication 7, **caractérisé en ce que** le mouvement de la plaque d'appui (27) est guidé par une membrane (30).

9. Dispositif selon la revendication 8, **caractérisé en ce que** la membrane (30) est fixée sur un boîtier (1a, 16).

10. Dispositif selon la revendication 9, **caractérisé en ce que** la membrane (30) délimite une chambre de pompe avec une partie (la) de boîtier.

11. Utilisation du dispositif selon l'une quelconque des revendications 1 à 10 pour pomper du sang.

Fig.1

Fig.2

Fig.3

Fig.4

Fig.5

Fig.6

Fig.7